(19) Europäisches Patentamt
European Patent Office
Office européen des brevets

(11) Veröffentlichungsnummer: **0 443 164 A1**

(12) **EUROPÄISCHE PATENTANMELDUNG**

(21) Anmeldenummer: **90124693.4**

(22) Anmeldetag: **19.12.90**

(51) Int. Cl.5: **C07D 285/12**, A01N 43/82

(30) Priorität: **20.02.90 DE 4005225**

(43) Veröffentlichungstag der Anmeldung:
**28.08.91 Patentblatt 91/35**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Förster, Heinz, Dr.**
**Am Eckbusch 47**
**W-5600 Wuppertal 1(DE)**
Erfinder: **Diehr, Hans-Joachim, Dr.**
**Höhe 35**
**W-5600 Wuppertal 11(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 145**
**W-5060 Bergisch-Gladbach 2(DE)**
Erfinder: **Schmidt, Robert R., Dr.**
**Im Waldwinkel 110**
**W-5060 Bergisch Gladbach 2(DE)**

(54) **Chlorfluormethyl-thiadiazolyloxyessigsäureamide.**

(57) Die Erfindung betrifft neue Chlorfluormethyl-thiadiazolyloxyessigsäureamide der allgemeinen Formel (I),

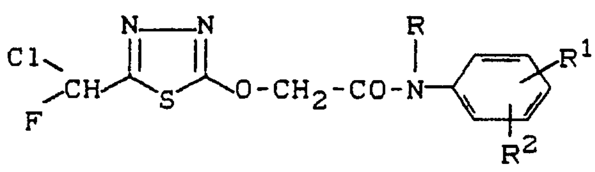

(I)

in welcher

R für geradkettiges oder verzweigtes Alkyl mit 2 bis 4 Kohlenstoffatomen steht und

$R^1$ und $R^2$ gleich oder verschieden sind und jeweils für Wasserstoff, Cyano, Nitro, Halogen oder für jeweils geradkettiges oder verzweigtes und gegebenenfalls durch Halogen substituiertes Alkyl, Alkoxy oder Alkylthio mit jeweils 1 bis 4 Kohlenstoffatomen stehen,

ein Verfahren und neue Zwischenprodukte zu ihrer Herstellung sowie ihre Verwendung als Herbizide.

EP 0 443 164 A1

Die Erfindung betrifft neue Chlorfluormethyl-thiadiazolyloxyessigsäureamide, ein Verfahren und neue Zwischenprodukte zu ihrer Herstellung sowie ihre Verwendung als Herbizide.

Es ist bekannt, daß bestimmte Heteroaryloxyessigsäureanilide, wie z.B. 2-(5-Chlor-1,3,4-thiadiazol-2-yl-oxy)-essigsäure-N-methyl-anilid, herbizide Eigenschaften aufweisen (vgl. EP-A 192117/LeA 23452). Weiter sind auch bestimmte Chlorfluormethyl-thiadiazolyloxyessigsäureamide als Herbizide bekanntgeworden (vgl. EP-A 148501/LeA 22781). Die herbizide Wirksamkeit bzw. die Verträglichkeit gegenüber Nutzpflanzen ist bei diesen bekannten Verbindungen jedoch nicht immer ganz zufriedenstellend.

Es wurden nun neue Chlorfluormethyl-thiadiazolyloxyessigsäureamide der allgemeinen Formel (I)

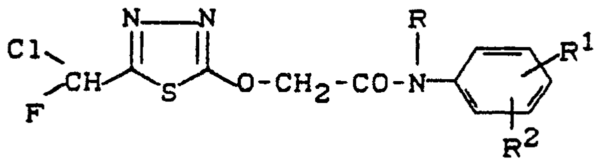

(I)

in welcher

R für geradkettiges oder verzweigtes Alkyl mit 2 bis 4 Kohlenstoffatomen steht und

$R^1$ und $R^2$ gleich oder verschieden sind und jeweils für Wasserstoff, Cyano, Nitro, Halogen oder für jeweils geradkettiges oder verzweigtes und gegebenenfalls durch Halogen substituiertes Alkyl, Alkoxy oder Alkylthio mit jeweils 1 bis 4 Kohlenstoffatomen stehen,

gefunden.

Weiter wurde gefunden, daß man die neuen Chlorfluormethyl-thiadiazolyloxyessigsäureamide der allgemeinen Formel (I) erhält, wenn man 2-Methylsulfinyl- oder 2-Methylsulfonyl-5-chlorfluormethyl-1,3,4-thiadiazol der allgemeinen Formel (II)

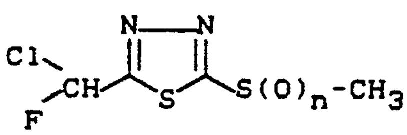

(II)

in welcher

n für die Zahlen 1 oder 2 steht,

mit Hydroxyessigsäureamiden der allgemeinen Formel (III)

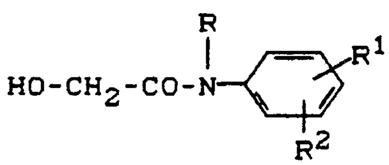

(III)

in welcher

R, $R^1$ und $R^2$ die oben angegebenen Bedeutungen haben,

gegebenenfalls in Gegenwart eines Säureakzeptors und gegebenenfalls in Gegenwart eines Verdünnungsmitels umsetzt.

Schließlich wurde gefunden, daß die neuen Chlorfluormethyl-thiadiazolyloxyessigsäureamide der allgemeinen Formel (I) starke und selektive herbizide Wirksamkeit zeigen.

Überraschenderweise zeigen die neuen Chlorfluormethylthiadiazolyloxyessigsäureamide der Formel (I) eine wesentlich bessere Kulturpflanzen-Verträglichkeit und stärkere Herbizidwirkung gegen verbreitete, schwer bekämpfbare Unkräuter als die oben genannte bekannte Verbindung.

Die Erfindung betrifft vorzugsweise Verbindungen der Formel (I), in welcher

R für Ethyl, Propyl, Isopropyl, Butyl, Isobutyl oder sec-Butyl steht,

$R^1$ für Wasserstoff, Cyano, Nitro, Fluor, Chlor, Brom, Methyl, Ethyl, Trifluormethyl, Methoxy, Ethoxy, Difluormethoxy, Trifluormethoxy, Chlordifluormethoxy, Methylthio, Ethylthio oder Trifluormethylthio steht, und

$R^2$ für Wasserstoff, Fluor, Chlor, Brom, Methyl, Ethyl, Trifluormethyl, Methoxy oder Ethoxy steht.

Die Erfindung betrifft insbesondere Verbindungen der Formel (I), in welcher

R für Ethyl oder Isopropyl steht,

$R^1$ für Wasserstoff, Fluor, Chlor, Methyl oder Methoxy steht und

$R^2$ für Wasserstoff, Fluor, Chlor oder Methyl steht.

Verwendet man beispielsweise 2-Methylsulfonyl-5-chlorfluormethyl-1,3,4-thiadiazol und Hydroxyessigsäure-N-ethyl-anilid als Ausgangsstoffe, so läßt sich der Reaktionsablauf beim erfindungsgemäßen Verfahren durch das folgende Formelschema darstellen:

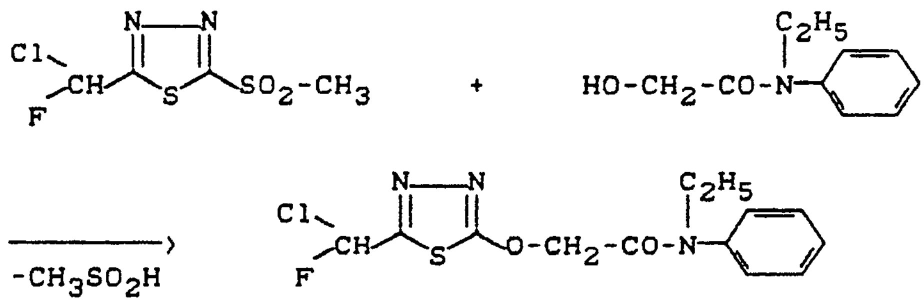


Die als Ausgangsstoffe zu verwendenden Verbindungen der Formel (II) - 2-Methylsulfinyl- und 2-Methylsulfonyl-5-chlorfluormethyl-1,3,4-thiadiazol - sind noch nicht aus der Literatur bekannt und sind Gegenstand der vorliegenden Erfindung.

Man erhält die neuen Verbindungen der Formel (II), wenn man 2-Methylthio-5-chlorfluormethyl-1,3,4-thiadiazol der Formel (IV)

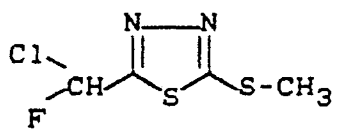

(IV)

mit einem Oxidationsmittel, wie z.B. Chlor (-Wasser) oder Hydrogenperoxid, gegebenenfalls in Gegenwart eines Katalysators, wie z.B. Natriumwolframat, und gegebenenfalls in Gegenwart von Verdünnungsmitteln, wie z.B. Wasser und/oder Essigsäure, bei Temperaturen zwischen 0° C und 100° C umsetzt.

Das als Zwischenprodukt benötigte 2-Methylthio-5-chlorfluormethyl-1,3,4-thiadiazol der Formel (IV) ist ebenfalls noch nicht aus der Literatur bekannt und Gegenstand der vorliegenden Erfindung.

Man erhält die neue Verbindung der Formel (IV), wenn man Chlorfluoressigsäure mit Dithiocarbazinsäure-methylester in Gegenwart von Phosphorylchlorid ($POCl_3$) bei Temperaturen zwischen -20° C und +120° C umsetzt (vgl. deutsche Patentanmeldung P 4 003 436.4 vom 06.02.1990).

Die beim erfindungsgemäßen Verfahren zur Herstellung von Verbindungen der Formel (I) weiter als Ausgangsstoffe zu verwendenden Hydroxyessigsäureamide sind durch die Formel (III) allgemein definiert.

In Formel (III) haben R, $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R, $R^1$ und $R^2$ angegeben wurden.

Als Beispiele für die Ausgangsstoffe der Formel (III) seien genannt:

N-Ethyl- und N-Isopropyl-hydroxyessigsäure-anilid, -2-fluor-anilid, -3-fluor-anilid, -4-fluor-anilid, -3,4-difluor-anilid, -2,4-difluor-anilid, -2,6-difluor-anilid, -2-chlor-anilid, -3-chlor-anilid, -4-chlor-anilid, -2,4-dichlor-anilid, -3,4-dichlor-anilid, -2,6-dichlor-anilid, -2,5-dichlor-anilid, -3,5-dichlor-anilid, -2-chlor-6-fluor-anilid, -3-brom-anilid, -4-brom-anilid, -3-cyano-anilid, -4-cyano-anilid, -3-nitro-anilid, -4-nitro-anilid, -2-methyl-anilid, -3-methyl-anilid, -4-methyl-anilid, -2,3-dimethyl-anilid, -2,4-dimethyl-anilid, -2,5-dimethyl-anilid, -3,4-dimethyl-anilid, -2-methyl-5-nitro-anilid,-2-chlor-5-methyl-anilid, -5-chlor-2-methyl-anilid, -2-chlor-6-methyl-anilid, -3-ethyl-anilid, -4-ethyl-anilid, -3-trifluormethyl-anilid, -4-trifluormethyl-anilid, -2-trifluormethyl-anilid, -2-methoxy-anilid, -3-methoxy-anilid, -3-ethoxy-anilid und -4-ethoxy-anilid.

Die Hydroxyessigsäureamide der Formel (III) sind bekannt und/oder können nach an sich bekannten Verfahren hergestellt werden (vgl. EP-A 37 526, US-P 4 509 971, US-P 4 645 525, US 4 334 073, EP-A 348737, DE-OS 3819477).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei

praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon, Hexamethylphosphorsäuretriamid, Alkohole, wie z.B. Methanol, Ethanol, Propanol, Isopropanol und Isobutanol, und Wasser.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z.B. Lithium-, Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +80 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in monokotylen und dikotylen Kulturen, vor allem im Vorauflauf- und Vorsaateinarbeitungs-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen

in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl]-aminosulfonyl}-benzoat (CHLORIMURON); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 1-Methyl-3-phenyl-5-(3-trifluormethylphenyl)-4-pyridon (FLURIDONE); 2-[5-Methyl-5-(1-methylethyl)-4-oxo-2-imidazolin-2-yl]-3-chinolincarbonsäure (IMAZAQUIN); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 1-(3-Trifluormethyl-phenyl)-4-methylamino-5-chlor-6-pyridazon (NORFLURAZON); 3-(Ethoxycarbonylaminophenyl)-N-(3'-methylphenyl)-carbamat(PHENMEDIPHAM); 0-(6-Chlor-3-phenyl-pyridazin-4-yl)-S-octylthiocarbonat (PYRIDATE); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden,

Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

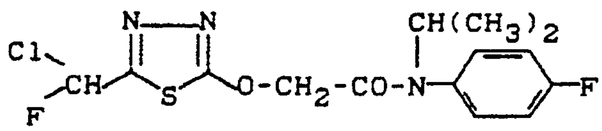

Eine Mischung aus 4,3 g (20 mMol) 2-Methylsulfinyl-5-chlorfluormethyl-1,3,4-thiadiazol, 4,2 g (20 mMol) N-Isopropyl-hydroxyessigsäure-4-fluor-anilid und 60 ml Aceton wird auf 0 °C abgekühlt und mit 1,0 g (24 mMol) Lithiumhydroxid-Hydrat versetzt. Das Reaktionsgemisch wird 5 Stunden bei 0 °C und 12 Stunden bei 20 °C gerührt. Dann werden 10 ml Essigsäure und 150 ml Wasser langsam dazugegeben. Das nach weiterem mehrstündigem Rühren kristallin angefallene Produkt wird durch Absaugen isoliert.

Man erhält 5,8 g (80% der Theorie) 2-(5-Chlorfluormethyl-1,3,4-thiadiazol-2-yl-oxy)-essigsäure-N-isopropyl-(4-fluor-phenyl-amid) vom Schmelzpunkt 103 °C.

Analog Beispiel 1 und entsprechend der allgemeinen Beschreibung des erfindungsgemäßen Herstellungsverfahrens können beispielsweise auch die weiteren in der nachstehenden Tabelle 1 aufgeführten Verbindungen der Formel (I) hergestellt werden.

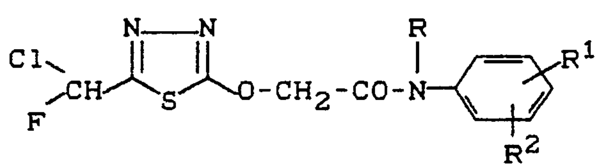

(I)

Tabelle 1: Beispiele für die Verbindungen der Formel (I)

| Bsp.-Nr. | R | $R^1$ | $R^2$ | Schmelzpunkt (°C) |
|---|---|---|---|---|
| 2 | $C_2H_5$ | H | H | 75 |
| 3 | $CH(CH_3)_2$ | (4-)Cl | H | 85 |
| 4 | $C_2H_5$ | (4-)Cl | H | |
| 5 | $C_2H_5$ | (3-)Cl | H | |
| 6 | $CH(CH_3)_2$ | (3-)Cl | H | |
| 7 | $C_2H_5$ | (3-)$CH_3$ | H | |
| 8 | $CH(CH_3)_2$ | (4-)$CH_3$ | H | 125 |
| 9 | $CH(CH_3)_2$ | (3-)$CH_3$ | H | |
| 10 | $C_2H_5$ | (4-)F | H | |
| 11 | $C_2H_5$ | (3-)F | H | |
| 12 | $C_2H_5$ | (3-)F | (4-)F | |
| 13 | $CH(CH_3)_2$ | (3-)F | (4-)F | |
| 14 | $C_2H_5$ | (2-)F | (5-)F | |
| 15 | $CH(CH_3)_2$ | (2-)F | (5-)F | |
| 16 | $C_2H_5$ | (3-)F | (5-)F | |
| 17 | $CH(CH_3)_2$ | (3-)F | (5-)F | |
| 18 | $C_2H_5$ | (2-)F | (4-)F | |

Tabelle 1 - Fortsetzung

| Bsp.-Nr. | R | $R^1$ | $R^2$ | Schmelzpunkt (°C) |
|---|---|---|---|---|
| 19 | $CH(CH_3)_2$ | (2-)F | (4-)F | |
| 20 | $C_2H_5$ | (3-)Cl | (4-)F | |
| 21 | $CH(CH_3)_2$ | (3-)Cl | (4-)F | |
| 22 | $C_2H_5$ | (4-)$CH_3$ | H | |
| 23 | $CH(CH_3)_2$ | (3-)$OCH_3$ | H | |
| 24 | $C_2H_5$ | (3-)$OCH_3$ | H | |
| 25 | $CH(CH_3)_2$ | (4-)$OCH_3$ | H | |
| 26 | $C_2H_5$ | (4-)$OCH_3$ | H | |
| 27 | $CH(CH_3)_2$ | (2-)$CH_3$ | (4-)$CH_3$ | |
| 28 | $C_2H_5$ | (2-)$CH_3$ | (4-)$CH_3$ | |
| 29 | $CH(CH_3)_2$ | (3-)$CH_3$ | (4-)$CH_3$ | |
| 30 | $C_2H_5$ | (3-)$CH_3$ | (4-)$CH_3$ | |
| 31 | $CH(CH_3)_2$ | (3-)$CH_3$ | (5-)$CH_3$ | |
| 32 | $C_2H_5$ | (3-)$CH_3$ | (5-)$CH_3$ | |

Ausgangsstoffe der Formel (II):

Beispiel (II-1)

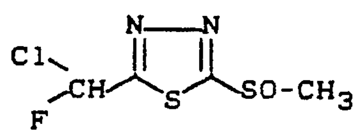

In eine Mischung aus 64 g (0,32 Mol) 2-Methylthio-5-chlorfluormethyl-1,2,4-thiadiazol und 500 ml Wasser wird bei 0° C bis 5° C ein kräftiger Chlorgas-Strom eingeleitet. Es bildet sich eine klare Lösung, aus der das Produkt allmählich kristallin ausfällt. Etwa 20 Minuten nach dem Beginn der Kristallisation wird das Einleiten von Chlor beendet, das Produkt durch Absaugen isoliert und aus Isopropanol umkristallisiert.

Man erhält 35 g (51% der Theorie) 2-Methylsulfinyl-5-chlorfluormethyl-1,3,4-thiadiazol vom Schmelzpunkt 58° C.

Beispiel (II-2)

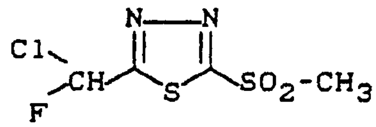

Eine Mischung aus 50 g (0,25 Mol) 2-Methylthio-5-chlorfluormethyl-1,3,4-thiadiazol, 100 ml Essigsäure und 0,5 g Natriumwolframat wird auf 30 °C bis 40 °C erwärmt und unter Rühren werden 50 ml einer 35%igen wäßrigen Hydrogenperoxidlösung (0,51 Mol $H_2O_2$) tropfenweise dazugegeben. Die Reaktionsmischung wird 15 Stunden bei 30 °C bis 40 °C und dann noch 2 Stunden bei 50 °C gerührt. Nach Verdünnen mit Eiswasser auf etwa das doppelte Volumen wird 3x mit je 100 ml Methyl-tert-butylether extrahiert; die vereinigten Extrakte werden mit Natriumhydrogensulfit-Lösung und dann mit Wasser gewaschen, mit Natriumsulfat getrocknet und filtriert. Vom Filtrat wird das Lösungsmittel im Wasserstrahlvakuum sorgfältig abdestilliert.

Man erhält 46,0 g (80% der Theorie) 2-Methylsulfonyl-5-chlorfluormethyl-1,3,4-thiadiazol als öligen Rückstand.
Brechungsindex: $n_D^{20}$ = 1,5278.

Ausgangsverbindung der Formel (IV)

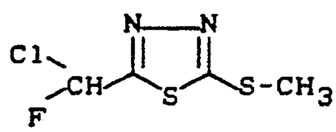

65 g (0,42 Mol) Phosphorylchlorid ($POCl_3$) werden unter Rühren zu einer auf 10 °C abgekühlten Mischung aus 11,3 g (0,10 Mol) Chlorfluoressigsäure und 12,2 g (0,10 Mol) Dithiocarbazinsäure-methylester tropfenweise gegeben. Das Reaktionsgemisch wird 30 Minuten bei 20 °C gerührt und dann bis zum Ende der Gasentwicklung auf 80 °C erhitzt. Nach Einengen im Vakuum wird der Rückstand mit Eiswasser verrührt und mit Chloroform geschüttelt. Von der organischen Phase wird dann das Lösungsmittel im Vakuum sorgfältig abdestilliert.

Man erhält 17,3 g (87% der Theorie) 2-Methylthio-5-chlorfluormethyl-1,3,4-thiadiazol als öligen Rückstand.
Brechungsindex: $n_D^{20}$ = 1,5776;
Siedepunkt: 90-91 °C/2 mbar.

Anwendungsbeispiel

Im folgenden Anwendungsbeispiel wird die Verbindung nachstehender Formel als Vergleichssubstanz verwendet:

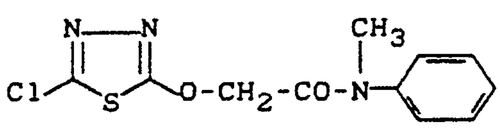

(A)

2-(5-Chlor-1,3,4-thiadiazol-2-yl-oxy)-essigsäure-N-methyl-anilid
(bekannt aus EP-A 192 117).

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit ebenso wie in der Nutzpflanzenselektivität gegenüber dem Stand der Technik zeigt in diesem Test z.B. die Verbindung gemäß Herstellungsbeispiel (1).

## Patentansprüche

1. Chlorfluormethyl-thiadiazolyloxyessigsäureamide der allgemeinen Formel (I),

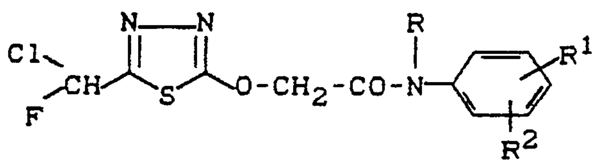

(I)

in welcher

R für geradkettiges oder verzweigtes Alkyl mit 2 bis 4 Kohlenstoffatomen steht und

$R^1$ und $R^2$ gleich oder verschieden sind und jeweils für Wasserstoff, Cyano, Nitro, Halogen oder für jeweils geradkettiges oder verzweigtes und gegebenenfalls durch Halogen substituiertes Alkyl, Alkoxy oder Alkylthio mit jeweils 1 bis 4 Kohlenstoffatomen stehen.

2. Chlorfluormethyl-thiadiazolyloxyessigsäureamide der allgemeinen Formel (I) gemäß Anspruch 1, dadurch gekennzeichnet, daß darin

R für Ethyl, Propyl, Isopropyl, Butyl, Isobutyl oder sec-Butyl steht,

$R^1$ für Wasserstoff, Cyano, Nitro, Fluor, Chlor, Brom, Methyl, Ethyl, Trifluormethyl, Methoxy, Ethoxy, Difluormethoxy, Trifluormethoxy, Chlordifluormethoxy, Methylthio, Ethylthio oder Trifluormethylthio steht, und

$R^2$ für Wasserstoff, Fluor, Chlor, Brom, Methyl, Ethyl, Trifluormethyl, Methoxy oder Ethoxy steht.

3. Chlorfluormethyl-thiadiazolyloxyessigsäureamide der allgemeinen Formel (I) gemäß Anspruch 1, dadurch gekennzeichnet, daß darin

R für Ethyl oder Isopropyl steht,

$R^1$ für Wasserstoff, Fluor, Chlor, Methyl oder Methoxy steht und

$R^2$ für Wasserstoff, Fluor, Chlor oder Methyl steht.

4. 2-(5-Chlorfluormethyl-1,3,4-thiadiazol-2-yl-oxy)-essigsäure-N-isopropyl-(4-fluor-phenyl-amid) der Formel (1)

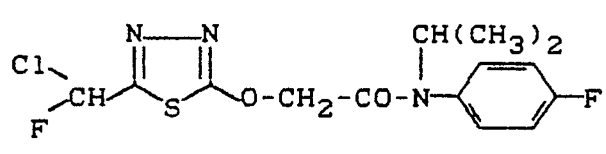

(1).

5. Verfahren zur Herstellung von Chlorfluormethylthiadiazolyloxyessigsäureamiden der Formel (I) gemäß Anspruch 1, dadurch gekennzeichnet, daß man 2-Methylsulfinyl- oder 2-Methylsulfonyl-5-chlor-fluormethyl-1,3,4-thiadiazol der allgemeinen Formel (II)

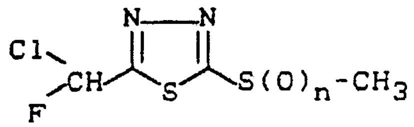

(II)

in welcher

n für die Zahlen 1 oder 2 steht,

mit Hydroxyessigsäureamiden der allgemeinen Formel (III)

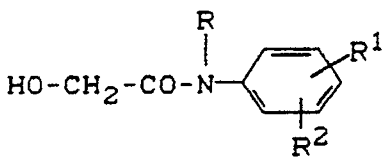

(III)

in welcher

R, $R^1$ und $R^2$ die oben angegebenen Bedeutungen haben,

gegebenenfalls in Gegenwart eines Säureakzeptors und gegebenenfalls in Gegenwart eines Verdünnungsmitels umsetzt.

6. Herbizide Mittel, gekennzeichnet durch einen Gehalt an mindestens einem Chlorfluormethyl-thiadiazolyloxyessigsäureamid der Formel (I) gemäß Anspruch 1.

7. Verfahren zur Bekämpfung von Unkräutern, dadurch gekennzeichnet, daß man Chlorfluormethylthiadiazolyloxyessigsäureamide der Formel (I) gemäß Anspruch 1 auf die Unkräuter oder ihren Lebensraum einwirken läßt.

8. Verwendung von Chlorfluormethyl-thiadiazolyoxyessigsäureamiden der Formel (I) gemäß Anspruch 1 zur Bekämpfung von Unkräutern.

9. Verfahren zur Herstellung von herbiziden Mitteln, dadurch gekennzeichnet, daß man Chlorfluormethylthiadiazolyloxyessigsäureamide der Formel (I) gemäß Anspruch 1 mit Streckmitteln und/oder oberflächenaktiven Mitteln vermischt.

10. 2-Methylsulfinyl- und 2-Methylsulfonyl-5-chlorfluormethyl-1,3,4-thiadiazol der allgemeinen Formel (II)

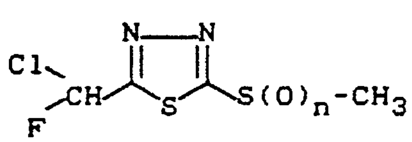

(II)

in welcher

n für die Zahlen 1 oder 2 steht.

Europäisches Patentamt

# EUROPÄISCHER RECHERCHENBERICHT

Nummer der Anmeldung

EP 90124693.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A1 - 0 348 736 (BAYER AG) * Ansprüche 1,4-10 * -- | 1,5-10 | C 07 D 285/12<br>A 01 N 43/82 |
| A | EP - A1 - 0 300 344 (BAYER AG) * Ansprüche 1,4-8 * -- | 1,5-9 | |
| P,A | DE - A1 - 3 838 432 (BAYER AG) * Ansprüche 1,5-9 * ---- | 1,5-10 | |

RECHERCHIERTE SACHGEBIETE (Int Cl 5)

C 07 D 285/00

Der vorliegende Recherchenbericht wurde für alle Patentansprüche erstellt.

| Recherchenort | Abschlußdatum der Recherche | Prüfer |
|---|---|---|
| WIEN | 20-03-1991 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument